# EUROPÄISCHE PATENTSCHRIFT

(11) **EP 3 872 083 B1**
(45) Veröffentlichungstag und Bekanntmachung des Hinweises auf die Patenterteilung: **14.06.2023**
(21) Anmeldenummer: 21163126.2
(22) Anmeldetag: 27.02.2017
(51) Int. Cl.: C07F 15/00, C07C 211/63, B01J 23/42, C01G 55/00, B01J 35/04, B01J 37/03

(54) **EDELMETALLVERBINDUNGEN**
PRECIOUS METAL COMPOUNDS
COMPOSÉS EN MÉTAUX PRÉCIEUX

(30) Priorität: 26.02.2016 EP 16157675
(43) Veröffentlichungstag der Anmeldung: 01.09.2021
(62) Teilanmeldung aus: 17706520.8
(73) Patentinhaber: UMICORE AG & Co. KG, 63457 Hanau-Wolfgang (DE)
(72) Erfinder: WOERNER, Eileen, 61130 Nidderau (DE); EBERT, Timo, 63589 Linsengericht (DE); LENNARTZ, Michael, 60435 Frankfurt (DE); KARCH, Ralf, 63801 Kleinostheim (DE); DOPPIU, Angelino, 63500 Seligenstadt (DE); RIVAS NASS, Andreas, 64625 Bensheim (DE); FREY, Annika, 63457 Hanau (DE)
(74) Vertreter: Clauswitz, Kai-Uwe Wolfram

(56) Entgegenhaltungen:
- EP-A2- 1 175 948
- WO-A1-2008/151731
- WO-A1-2015/168327
- WO-A2-2008/145386
- CN-A- 103 864 855
- CN-A- 103 880 889
- DATABASE CAPLUS [Online] Chemical Abstracts; 1. Januar 1984 (1984-01-01), Kulpina E. G. ET AL: "Interaction of rhodium(III) with cetylpyridinium cations in alkaline media", XP055816748, gefunden im STN Database accession no. 1985:16500
- FORTEA-PÉREZ FRANCISCO RAMÓN ET AL: "Structural insight into the reaction mechanism of Pd-catalyzed nitrile hydration: Trapping the [Pd(H2O)4]2+cation through a supramolecular complex", INORGANICA CHIMICA ACTA, ELSEVIER BV, NL, Bd. 443, 8. Januar 2016 (2016-01-08), Seiten 267-273, XP029406224, ISSN: 0020-1693, DOI: 10.1016/J.ICA.2016.01.002
- ZHENGZHENG YANG ET AL: "Size-dependent CO and propylene oxidation activities of platinum nanoparticles on the monolithic Pt/TiO 2 -YO x diesel oxidation catalyst under simulative diesel exhaust conditions", CATALYSIS SCIENCE & TECHNOLOGY, Bd. 5, Nr. 4, 2015, Seiten 2358-2365, XP055295839, United Kingdom ISSN: 2044-4753, DOI: 10.1039/C4CY01384K

## Beschreibung

Bei der Abscheidung von Platinmetallen auf Trägermaterialien, wie z.B. Oxidpartikeln, werden an die platinhaltigen Platin-Ausgangsverbindungen mehrere Anforderungen gestellt.

Diese sollen vorteilhaft in basischen Lösungen mit einem Metallgehalt von mindestens etwa 10% (wie z.B. 9 bis 11%) hergestellt werden können, halogenfrei oder zumindest halogenarm sein, mit Ausnahme von Kohlenstoff, Wasserstoff, Sauerstoff, und Stickstoff keine Fremdelemente enthalten, einen möglichst niedrigen Stickstoffgehalt aufweisen, um Stickoxidemissionen zu minimieren. Zudem sollen sie durch Veränderung des pH Werts möglichst vollständig ausgefällt werden können.

Kulpina et al., "Interaction of rhodium(III) withy cetylpyridinium cations in alkaline media", Chemical Abstracts, 1.Januar 1984 zeigt Rhodium(III)-Salze, die auch Tetraalkylammoniumverbindungen enthalten können und quaternäre Pyridiniumkationen aufweisen.

Fortea-Pérez et al., « Structural insight into the reation mechanism of Pdcatalyzed nitrile hydration: Trapping the [Pd(H2O)4]2+ cation through supramolecular complex, Inorganica Chimica Acta, Bd. 443, 8.1.2016, Seiten 267-273 zeigt [Pd(H₂O)₄]²⁺-Komplexe als Nitril-Hydratisierungs-Katalysatoren.

EP 1175948 A2 zeigt geträgerte Platinverbindungen, Palladiumteilchen sowie Rhodiumteilchen als Nanoteilchen sowie die Verbindungen [(H₃N-Et)²⁺ [Pt(OH)₆]⁻, [(H₃N-CH₂CH₂OH)]₂⁺[Pt(OH)₆]²⁻, [(NH₃)₄[Pd(II)(NO₃)₂] und Trinitratonitrosyl-Ruthenium.

CN 103864855 A offenbart die Verbindung [HO-CH₂-CH₂-NH₃][Pt(OH)₆] (Pt-EAH) sowie Verfahren zur Herstellung aus H₂Pt(OH)₆ und EA in Wasser und die Verwendung von Pt-EAH als Precursorverbindung zur Herstellung geträgerter Katalysatoren.

CN 103880889 A zeigt die Verbindung (Pt-EAH) sowie Verfahren zur Herstellung aus H₂Pt(OH)₆ und EA und die Verwendung von Pt-EAH als Precursorverbindung zur Herstellung geträgerter Katalysatoren mit niedrigem Chlorid- und Nitratgehalt.

Zhengzheng Yang et al., Catalysis Science and Technology Bd. 5, Nr. 4, 2015, Seiten 2358-2365 offenbart die Verwendung von (Pt-EAH) als Precursorverbindung zur Herstellung geträgerter Platinkatalysatoren zur Abgasbehandlungen von Dieselmotoren.

WO 2008/151731 zeigt Tetramethylammonium-Hexahydroxoplatinat sowie dessen Verwendung als Precursor zur Herstellung heterogener Katalysatoren durch Beschichten eines Tragkörpers, durch Imprägnieren des Formkörpers, sowohl mit einer sauren wässrigen Metallsalzlösung als auch mit einer basischen wässrigen Lösung, wobei die Basenlösung die Metallkomponente ausfällt.

WO 2008/151731 offenbart Tetraalkylammonium Hexahydroxoplatinat bzw. Tetramethylammonium-Hexahydroxoplatinat und bezieht sich weiter auf die Metalle Palladium und Rhodium.

WO 2015/168327 zeigt Tetramethylammonium-Hexahydroxoplatinat sowie [(Ethanolamine-H)₂Pt(OH)_{6]} und zeigt ausserdem einen geträgerten Katalysator aus Trinitratonitrosyl-Ruthenium auf Kohlenstoff.

Diese Vorgaben werden von Tetraalkylammonium- Tetra- oder Hexahydroxometallaten der Formel (N(Alkyl)₄)_{y}[M(OH)ₓ], wobei
M ein Metall ausgewählt aus der Gruppe bestehend aus Rhodium oder Palladium ist;
Alkyl eine Alkylgruppe mit einem bis 6 Kohlenstoffatomen ist;
Y 1, 2 oder 3 ist; und
X 4 oder 6 ist.

Alkyl kann insbesondere Alkyl eine Alkylgruppe ausgewählt aus der Gruppe bestehend aus Ethyl, Propyl, Isopropyl, n-Butyl, Isobutyl, tert.-Butyl, n-Pentyl, sec-Pentyl, 3-Pentyl, 2-Methylbutyl, Isopentyl, 3-Methylbut-2-yl, 2-Methylbut-2-yl, Neopentyl, n-Hexyl, 3-Methylpentyl, Isohexyl, Neohexyl, 2,3,-Dimethylbutyl oder deren Kombinationen sein. Die Alkylgruppen können gleich oder verschieden sein, vorteilhaft sind sie gleich.

Entsprechend der Definition der Variablen M in der Formel von Anspruch 1 beziehen sich alle nachfolgenden Offenbarungen, die generisch von "Metall" sprechen, spezifisch und ausschliesslich auf entweder Rhodium oder Palladium als Metal.

Der Gehalt an Halogen (insbesondere Chlor) wird über den Chlorgehalt der verwendeten Edukte eingestellt.

Dabei handelt es sich insbesondere um die folgenden Einzelverbindungen der allgemeinen Formel (N(Alkyl)₄)_{y}[M(OH)ₓ], wobei Alkyl, M und x die folgenden Bedeutungen gemäß Formel 1 aufweisen:

| Nummer | M | X | Y | Alkyl |
|---|---|---|---|---|
| 1 | Rhodium | 6 | 3 | Ethyl |
| 2 | Rhodium | 6 | 3 | Propyl |
| 3 | Rhodium | 6 | 3 | Isopropyl |
| 4 | Rhodium | 6 | 3 | n-Butyl |
| 5 | Rhodium | 6 | 3 | Isobutyl |
| 6 | Rhodium | 6 | 3 | tert.-Butyl |
| 7 | Rhodium | 6 | 3 | n-Pentyl |
| 8 | Rhodium | 6 | 3 | sec-Pentyl |
| 9 | Rhodium | 6 | 3 | 3-Pentyl |
| 10 | Rhodium | 6 | 3 | 2-Methylbutyl |
| 11 | Rhodium | 6 | 3 | Isopentyl |
| 12 | Rhodium | 6 | 3 | 3-Methylbut-2-yl |
| 13 | Rhodium | 6 | 3 | 2-Methylbut-2-yl |
| 14 | Rhodium | 6 | 3 | Neopentyl |
| 15 | Rhodium | 6 | 3 | n-Hexyl |
| 16 | Rhodium | 6 | 3 | 3-Methylpentyl |
| 17 | Rhodium | 6 | 3 | Isohexyl |
| 18 | Rhodium | 6 | 3 | Neohexyl |
| 19 | Rhodium | 6 | 3 | 2,3,-Dimethylbutyl |
| 20 | Palladium | 4 | 1 bzw. 2 | Ethyl |
| 21 | Palladium | 4 | 1 bzw. 2 | Propyl |
| 22 | Palladium | 4 | 1 bzw. 2 | Isopropyl |
| 23 | Palladium | 4 | 1 bzw. 2 | n-Butyl |
| 24 | Palladium | 4 | 1 bzw. 2 | Isobutyl |
| 25 | Palladium | 4 | 1 bzw. 2 | tert.-Butyl |
| 26 | Palladium | 4 | 1 bzw. 2 | n-Pentyl |
| 27 | Palladium | 4 | 1 bzw. 2 | sec-Pentyl |
| 28 | Palladium | 4 | 1 bzw. 2 | 3-Pentyl |
| 29 | Palladium | 4 | 1 bzw. 2 | 2-Methylbutyl |
| 30 | Palladium | 4 | 1 bzw. 2 | Isopentyl |
| 31 | Palladium | 4 | 1 bzw. 2 | 3-Methylbut-2-yl |
| 32 | Palladium | 4 | 1 bzw. 2 | 2-Methylbut-2-yl |
| 33 | Palladium | 4 | 1 bzw. 2 | Neopentyl |
| 34 | Palladium | 4 | 1 bzw. 2 | n-Hexyl |
| 35 | Palladium | 4 | 1 bzw. 2 | 3-Methylpentyl |
| 36 | Palladium | 4 | 1 bzw. 2 | Isohexyl |
| 37 | Palladium | 4 | 1 bzw. 2 | Neohexyl |
| 38 | Palladium | 4 | 1 bzw. 2 | 2,3,-Dimethylbutyl |

Während Hexahydroxoplatinate eine gut definierte Struktur aufweisen ist die Struktur bei anderen Metallhydroxiden nicht immer eindeutig charakterisiert bzw. charakterisierbar.

So kann zum Beispiel die Verbindung gemäß der Erfindung im Falle von Rhodium die Struktur [N(Alkyl)₄]₃[Rh(OH)₆] aufweisen, wie beispielsweise im Fall von Rh-TEAH [N(CH₂CH₃)₄]₃[Rh(OH)₆].

Bei Palladium können auch Strukturen wie (N(Alkyl)₄)₂[Pd(OH)₄], aber auch (N(Alkyl)₄) [Pd(OH)₃(H₂O)], einzeln oder im Gemisch auftreten.

Im Allgemeinen können die Verbindungen auch in Strukturen wie (N(Alkyl)₄)ₛ [M(OH)ᵥ(H₂O)_{w}]^{u-v}, wobei u die Ladung des Metalls, v = 1 bis Koordinationszahl des Metalls, w = Koordinationszahl des Metalls -v und s = 1 bis u-v sein kann.

Die beschriebenen Verbindungen, Tetraalkylammonium- Tetra- bzw. Hexa-hydroxometallate der Formel (N(Alkyl)₄)_{y}[M(OH)ₓ], können durch Umsetzung von Hydroxoverbindungen des Metalls wie etwa Tetra- oder Hexahydroxosäuren der jeweiligen Metalle mit den jeweiligen Tetraalkylammoniumhydroxiden, gegebenenfalls in einem Reaktionsmedium erhalten werden.

Als Hydroxoverbindungen insbesondere geeignet sind im Fall von Rhodium Rhodiumhydroxid, bei Palladium Palladiumhydroxid. Diese Verbindungen können durch Zugabe von Metallsalzen zu einer Base gefällt und so erhalten werden. Als Basen geeignet sind Alkalimetallhydroxide bzw. deren wässrige Lösungen, aber auch die ohnehin verwendeten Tetraalkylammoniumhydroxide, wie speziell Tetraethylammoniumhydroxid. Ein Beispiel hierfür ist die Fällung von Palladiumhydroxid durch Zufügen einer Lösung von Palladiumnitrat oder Palladiumchlorid zu einer Lösung von Tetraethylammoniumhydroxid. Ist ein möglichst niedriger Halogengehalt gewünscht, so ist natürlich das Nitrat dem Halogenid vorzuziehen.

Diese Verbindungen sind nicht immer eindeutig charakterisierbar und können teilweise auch Aquo-Liganden aufweisen. Alle solchen Verbindungen sind hier kollektiv mit Hydroxoverbindungen eines Metalls wie etwa deren Tetra- oder Hexahydroxosäuren gemeint, als welche die Ausgangsverbindungen auch aufgefasst werden können. So wird Rhodiumhydroxid oft auch als H₃[Rh(OH)₆] beschrieben.

Die Hydroxoverbindungen eines Metalls wie etwa Tetra- oder Hexahydroxosäuren der jeweiligen Metalle können mit verschiedenen Halogengehalten, insbesondere Chlorgehalten, hergestellt werden. Diese Halogengehalte liegen meist bei 30.000 ppm oder weniger, oder 20.000 ppm oder weniger, oder 15.000 ppm oder weniger, insbesondere 10.000 ppm oder weniger, 5.000 ppm oder weniger oder 1.000 ppm oder weniger, wobei sich der Halogengehalt auf das Metall bezieht. Insbesondere handelt es sich bei dem Halogengehalt um einen Chlorgehalt, der in den oben angeführten Gehalten auftreten kann.

Es ist unkritisch, ob die Hydroxoverbindungen eines Metalls wie etwa Tetra-oder Hexahydroxosäuren der jeweiligen Metalle oder das jeweilige Tetraalkylammoniumhydroxid vorgelegt und der jeweilige Reaktionspartner zugegeben wird. Daher können die Hydroxoverbindungen eines Metalls wie etwa Tetra-oder Hexahydroxosäuren des gewünschten Metalls oder deren Lösung vorgelegt und ein Tetraalkylammoniumhydroxid mit dem gewünschten Alkylrest, gegebenenfalls in Lösung, zugegeben werden oder aber es kann das Tetraalkylammoniumhydroxid mit dem gewünschten Alkylrest oder dessen Lösung vorgelegt und die Hydroxoverbindungen des Metalls wie etwa Tetra-oder Hexahydroxosäuren des gewünschten Metalls, gegebenenfalls in Lösung, zugegeben werden.

Optional kann die Umsetzung in einem Reaktionsmedium erfolgen, welches vorteilhaft ein Lösemittel sein kann. Gut geeignet sind polare, protische Lösemittel.

Das Reaktionsmedium kann ausgewählt sein aus der Gruppe bestehend aus Wasser, ein oder mehrere Alkohole, eine oder mehrere Säuren und deren Mischungen. Insbesondere kann das Reaktionsmedium ein Lösemittel ausgewählt aus der Gruppe bestehend aus Wasser, Methanol, Ethanol, Propanol, Isopropanol, Butanol, 1-Methylbutanol, 2-Methylbutanol, tert.-Butanol, Ameisensäure, Essigsäure, Propionsäure und deren Kombinationen sein. Besonders geeignet als Reaktionsmedium ist Wasser; in der Praxis wird in diesem Fall üblicherweise demineralisiertes Wasser bzw. destilliertes Wasser verwendet.

Vorteilhaft wird das Reaktionsmedium vor Zugabe der Edukte vorgelegt. Es kann jedoch auch gleichzeitig mit einem Edukt vorgelegt werden, beispielsweise wenn ein Edukt oder beider Edukte als Lösung oder Suspension vorgelegt bzw. zugegeben wird, wie zum Beispiel eine Lösung von Tetraethylammoniumhydroxid.

Die Edukte werden für eine vorbestimmte Reaktionszeit und bei einer in einem vorbestimmten Bereich liegenden Reaktionstemperatur umgesetzt.

Im Allgemeinen ist eine Reaktionszeit von 10 Minuten bis 24 Stunden ausreichend, insbesondere eine Reaktionszeit von 20 Minuten bis 12 Stunden, 30 Minuten bis 6 Stunden, 45 Minuten bis 3 Stunden oder 45 Minuten bis 120 Minuten, oder 50 Minuten bis 70 Minuten. Meist ist eine Reaktionszeit von etwa einer Stunde ausreichend.

Die Reaktionstemperatur kann von 10°C bis 100°C, insbesondere 15°C bis 80°C oder 20°C bis 50°C oder 20°C bis 40°C betragen. Wird ein Reaktionsmedium eingesetzt, so liegt die Reaktionstemperatur maximal bei der Siedetemperatur des Reaktionsmediums. In der Praxis bewährt haben sich Temperaturen von 20°C bis 50°C.

Zur Herstellung der gewünschten Tetraalkylammonium- Tetra- oder Hexa-hydroxometallate der Formel (N(Alkyl)₄)_{y}[M(OH)ₓ] werden mindestens 0,5, insbesondere mindestens 1,7 Moläquivalente Tetraalkylammoniumhydroxid pro Mol der Hydroxoverbindungen des Metalls wie etwa Tetra- oder Hexahydroxosäuren des Metalls eingesetzt. Bewährt haben sich 0,5 Moläquivalente bis 17 Moläquivalente, 1,7 Moläquivalente bis 17 Moläquivalente, insbesondere 2 Moläquivalente bis 6 Moläquivalente Tetraalkylammoniumhydroxid pro Mol der Hydroxoverbindungen des Metalls wie etwa Tetra-oder Hexahydroxosäuren des Metalls. Ist das verwendete Metall M Palladium, so sind insbesondere 0,5 bis 2 Moläquivalente, insbesondere 1 bis 1,7 Moläquivalente Tetraalkylammoniumhydroxid vorteilhaft

Das gewünschte Produkt (Tetraalkylammonium- Tetra- oder Hexa-hydroxometallat der Formel (N(Alkyl)₄)_{y}[M(OH)ₓ] fällt bei Verwendung eines Reaktionsmediums als Lösung an, insbesondere als wässrige Lösung, wobei die Konzentration an Palladium oder Rhodium von 1 Gew.-% bis 15 Gew.-%, insbesondere 10 Gew.-% bis 15 Gew.-% beträgt.

Die Tetraalkylammonium- Tetra- oder Hexa-hydroxometallate der Formel (N(Alkyl)₄)_{y}[M(OH)ₓ] bzw. deren Zubereitungen können als Precursor zur Herstellung heterogener Katalysatoren (wie z.B. Autoabgaskatalysatoren), für den Einsatz in galvanischen Bädern, als Vorprodukt für die Herstellung homogener Katalysatoren oder auch selbst als homogener Katalysator verwendet werden. Ebenfalls geeignet sind diese zur Herstellung von Materialien für Brennstoffzellen.

Zur Herstellung eines heterogenen Katalysators kann eine Lösung eines Tetraalkylammonium- Tetra- oder Hexa-hydroxometallate der Formel (N(Alkyl)₄)_{y}[M(OH)ₓ] mit einem Trägermaterial wie einem Metalloxid wie zum Beispiel Aluminiumoxid oder Siliziumdioxid vermischt werden. Das Metalloxid, also Aluminiumoxid oder Siliziumdioxid, wird vorteilhaft in Form von Pulver oder Partikel eingesetzt.

Anschließend wird das Tetraalkylammonium- Tetra- oder Hexa-hydroxometallat der Formel (N(Alkyl)₄)_{y}[M(OH)ₓ] umgesetzt, d.h. einer chemischen Reaktion unterworfen, wobei eine Metallverbindung entsteht, gegebenenfalls ausfällt und sich zumindest teilweise auf der Oberfläche der Trägermaterialpartikel, also der Metalloxidpartikel, abscheidet. Die Abscheidung kann somit durch Auffällen, aber auch durch Adsorption erfolgen.

Unter bestimmten, geeigneten Bedingungen kann auch das elementare Metall abgeschieden werden. Hierzu muss ein Reduktionsmittel zugefügt werden, wie beispielsweise Wasserstoff, Hydrazin oder Ameisensäure. Dieses Verfahren ist insbesondere zur Herstellung von Materialien für Brennstoffzellen von Interesse.

Die Umsetzung und das Ausfällen der Metallverbindung kann durch Änderung des pH-Wertes erfolgen. Hierbei kann insbesondere eine Hydroxoverbindung des jeweiligen Metalls wie etwa die Tetrahydroxosäure oder Hexahydroxosäure des Metalls (also von Rhodium oder Palladium) ausgefällt und auf den Metalloxidpartikeln abgeschieden werden.

Durch Kalzinieren der so erhaltenen Metalloxidpartikel, auf denen die Metallverbindung abgeschieden wurde, kann in einem weiteren Verfahrensschritt elementares Metall auf den Partikeln des Trägermaterials erzeugt werden.

Sowohl Trägermaterial, auf dem die Metallverbindung abgeschieden wurde als auch Trägermaterial, auf dem die Metallverbindung durch Kalzinierung zu elementarem Metall umgesetzt wurde, können zur Herstellung von Autoabgaskatalysatoren verwendet werden.

Hierzu wird eine Beschichtungssuspension (auch als Washcoat bezeichnet) hergestellt, welche das Trägermaterial enthält. Hierbei handelt es sich wie oben beschrieben meist um Metalloxide, insbesondere also Aluminiumoxid oder Siliziumdioxid.

Mit dieser Beschichtungssuspension werden die Innenwände der Strömungskanäle der Tragkörper, welche zur Herstellung der Autoabgaskatalysatoren erforderlich sind, auf an sich bekannte Weise beschichtet, wie z.B. in EP-A1-1273344 beschrieben.

Die Tragkörper werden meist aus Metall oder Keramik hergestellt und können als Durchflußwabenkörper oder Wandflußfilter ausgestaltet sein. Hierbei handelt es sich im Allgemeinen um zylindrische Körper, die eine umlaufende Mantelfläche und zwei gegenüberliegende Stirnflächen aufweisen, wobei die Tragkörper von Strömungskanälen durchzogen sind, die zwischen beiden Stirnflächen verlaufen.

Weit verbreitet sind Katalysatorkörper mit rundem Querschnitt, elliptischem oder dreieckförmigem Querschnitt. Die Strömungskanäle weisen meist einen quadratischen Querschnitt auf und sind in einem engen Raster über den gesamten Querschnitt der Katalysatorkörper angeordnet. Je nach Anwendungsfall variiert die Kanal- beziehungsweise Zelldichte der Strömungskanäle meist zwischen 10 und 250 cm⁻². Für die Abgasreinigung von Personenkraftwagen werden heute noch oft Katalysatortragkörper mit Zelldichten von etwa 62 cm⁻² eingesetzt. Bei Wandflussfiltern sind diese alternierend verschlossen, so dass ein durchfließender Abgasstrom durch die Poren der Wandungen gelenkt wird, welche die Strömungskanäle bilden.

Nach dem Aufbringen der Beschichtungssuspension wird der beschichtete Wabenkörper gegebenenfalls weiteren Verfahrensschritten unterworfen, getrocknet und schließlich kalziniert. Wird Trägermaterial, auf dem eine Metallverbindung abgeschieden wurde, verwendet, so findet die Umwandlung in elementares Metall dann bei diesem Kalzinierungsschritt nach Beschichtung des Tragkörpers statt.

Wurde bereits ein nach Abscheidung der Metallverbindung kalziniertes Trägermaterial (wie z.B. Platin auf einem Aluminiumoxid-Trägermaterial) zur Herstellung der Beschichtungssuspension verwendet, so liegt bereits vor der Beschichtung des Tragkörpers elementares Metall auf dem Trägermaterial vor und die Kalzinierung des beschichteten Tragkörpers ist hinsichtlich der Umwandlung der abgeschiedenen Metallverbindung ohne Effekt.

### Beispiele

### Beispiel 1: Herstellung von Pt-TEAH (Tetraethylammonium-hexahydroxoplatinat) (nicht erfindungsgemäß)

In einem Reaktor mit einem Füllvolumen von fünf Litern wurden 758 g demineralisiertes Wasser vorgelegt und 1,47 kg wässrige Tetraethylammoniumhydroxidlösung mit einer Konzentration von 35 Gew.-% zugegeben, entsprechend etwas 2 mol pro mol Platin.

Unter Rühren wurden dann 894,74g Hexahydroxoplatinsäure mit einem Gehalt von 38 g Platin zugegeben. Es wurde anschließend bei einer Temperatur von ca. 20°C bis 30°C eine Stunde nachgerührt, wobei eine gelborange Lösung entstand, die über eine G4-Glasfritte filtriert und analysiert wurde.

Die Lösung enthielt einen Edelmetallgehalt von 10,9 Gew.-% Platin und einen pH-Wert von 13,9.

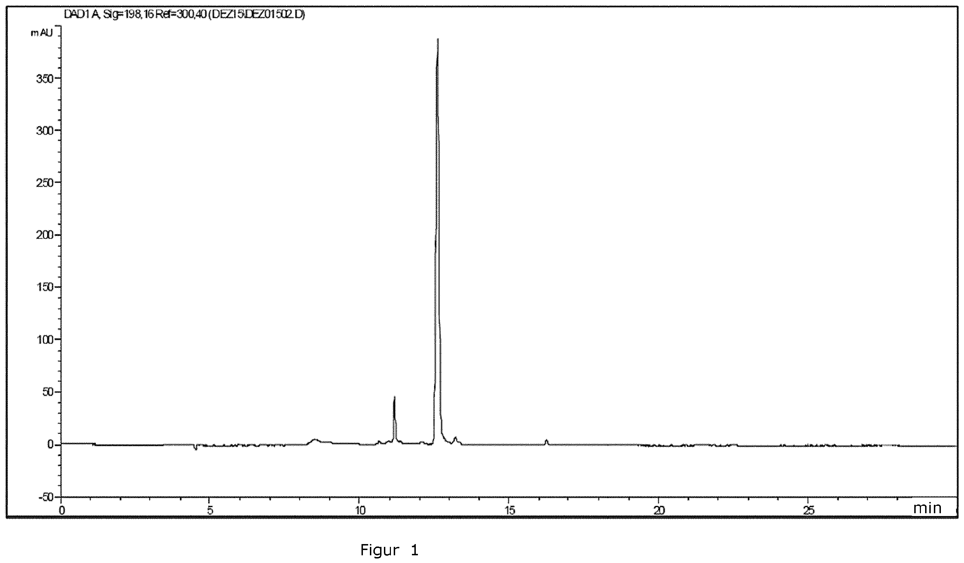

Das Produkt wurde auch durch Kapillarelektrophorese untersucht, das Ergebnis ist in Figur 1 gezeigt. Die Zusammensetzung ist charakteristisch für die Verbindung Pt-TEAH. Der Hauptpeak entspricht dem [Pt(OH)₆]²⁻ Komplex. Der kleinere, zweite Peak entspricht einem weiteren anionischen Pt-Komplex der unbekannten Zusammensetzung [Pt(OH)₆₋ₓ(NEt)ₓ]^{2-x} (mit x=1-2). Die prozentuale Verteilung beider Hauptkomponenten kann je nach Herstellmethode und Eduktqualität schwanken.

### Beispiel 2: Herstellung von Rh-TEAH

13,5 g Rhodiumhydroxid (RhOH₃) wurden in 10 g Wasser suspendiert und 61,594 g wässrige Tetraethylammoniumhydroxidlösung mit einer Konzentration von 35 Gew.-% in einem Schuss zugegeben und bei einer Temperatur von 22°C für eine Stunde nachgerührt, wobei sich nach etwa 30 Minuten eine braune Lösung bildete, die über eine G4-Glasfritte filtriert und analysiert wurde.

Die Lösung enthielt einen Edelmetallgehalt von 10 Gew.-% und einen pH-Wert von etwa 10 (Bestimmung mit Universalindikatorpapier).

### Beispiel 3: Herstellung von Pd-TEAH

### Herstellung des Hydroxids

866 g wässrige Tetraethylammoniumhydroxidlösung mit einer Konzentration von 35 Gew.-% wurden vorgelegt und innerhalb von 30 Minuten etwa 540 g einer Palladiumnitratlösung mit einem Palladiumgehalt von 130 g zugetropft, bis der pH-Wert 3,6 bis 3,7 betrug; die genaue Menge der Lösung ist abhängig vom Palladiumgehalt und kann abhängig davon schwanken. Während der Zugabe trat eine Wärmetönung auf, die zu einer Erwärmung auf 40°C führte.

Es wurde über Nacht für etwa 18 Stunden nachgerührt und über eine G4-Glasfritte filtriert. Der erhaltene Feststoff, das gefällte Hydroxid, wurde mit demineralisiertem Wasser gewaschen, bis das Filtrat sich dunkelbraun färbte, über Nacht im Vakuum getrocknet.

### Herstellung von Pd-TEAH

Anschließend wurde ein 1,0 Moläquivalent Tetraethylammoniumhydroxidlösung mit einer Konzentration von 35 Gew.-% vorgelegt, das getrocknete Filtrat zugegeben und eine Stunde bei einer Temperatur von 23°C gerührt. Anschließend wurde über eine G4-Glasfritte filtriert und getrocknet.

Die Ausbeute an (N(Alkyl)₄)_{y}[M(OH)ₓ] betrug 90 %, bezogen auf das eingesetzte Palladium.

Die Lösung enthielt einen Edelmetallgehalt von 10,2 Gew.-% und einen pH-Wert von etwa 11 (Bestimmung mit Universalindikatorpapier).

### Beispiel 4: Herstellung von Pt-TPAH (Tetrapropylammonium-hexahydroxoplatinat; Alkyl = n-Propyl) (nicht erfindungsgemäß)

Es wurde verfahren wie in Beispiel 1, allerdings wurde eine wässrige Lösung von Tetrapropylammoniumhydroxid (40 Gew.-%) eingesetzt und eine wässrige Lösung enthaltend 31,92 Gew.-% Hexahydroxoplatinsäure vorgelegt und für 180 Minuten bei 20°C bis 25°C gerührt, danach wurde auf 50°C erwärmt, weitere 5 ml der Tetrapropylammoniumhydroxidlösung zugegeben, über Nacht (ca. 18 Stunden) stehen gelassen und über eine G4-Glasfritte filtriert und 15 Minuten zentrifugiert. Die erhaltene klare Tetrapropylammonium-hexahydroxoplatinat Lösung wurde wies einen Platingehalt von 9,08% auf.

### Beispiel 5: Herstellung von Pt-TBAH (Tetrabutylammonium-hexahydroxoplatinat; Alkyl = n-Butyl) (nicht erfindungsgemäß)

Es wurde verfahren wie in Beispiel 4, allerdings wurde gefrorenes Tetrabutylammoniumhydroxid^{∗}30 H₂0 eingesetzt, eine wässrige Lösung enthaltend 39,3 Gew.-% Hexahydroxoplatinsäure vorgelegt und über Nacht (ca. 18 Stunden) bei 20°C bis 25°C gerührt und 15 Minuten zentrifugiert. Die erhaltene klare Tetrabutylammonium-hexahydroxoplatinat-Lösung wies eine dunkelgelbe Farbe auf.

Die Lösung enthielt einen Edelmetallgehalt von 9,8 Gew.-% und einen pH-Wert von etwa 10 (Bestimmung mit Universalindikatorpapier).

### Beispiel 6: Herstellung einer Beschichtungssuspension zur Herstellung eines heterogenen Katalysators (nicht erfindungsgemäß)

45 g Aluminiumoxid und 16 g eines Zeolithen (Zeolite-HSZ-0940NHA von Tosoh) wurden in Wasser suspendiert und für 15 Minuten gerührt. Anschließend wurde durch Zugabe von Tetraethylammoniumhydroxid der pH-Wert auf 10 eingestellt, dann wurden 0,81 g gelöstes Platin in Form einer löslichen Platinverbindung zugegeben und für 15 Minuten nachgerührt. Anschließend wurden 0,54 g Palladium in Form von Palladiumnitrat in Wasser zugegeben und erneut für 15 Minuten nachgerührt. Anschließend wurde mit Essigsäure der pH-Wert auf einen Wert von 5 eingestellt und 5 Minuten nachgerührt. Anschließend wurde der Gehalt an Edelmetall bestimmt, der noch in Wasser gelöst vorlag.

Vergleichsbeispiel 6a: Das Platin wurde in Form einer Ethanolamin-Hexahydroxoplatinatlösung in Wasser zugegeben. Es wurde festgestellt, dass nach Abschluss des Versuchs 10% der ursprünglichen Palladiumkonzentration und 20% der ursprünglichen Palladiumkonzentration gelöst vorlagen. Das restliche Edelmetall war auf den vorhandenen Träger (Aluminiumoxid und Zeolith) aufgefällt.

Beispiel 6b: Das Platin wurde in Form einer wässrigen Lösung von Tetraethylammonium-hexahydroxoplatinat (Pt-TEAH) zugegeben. Nach Abschluss des Versuchs wurde festgestellt, dass sich kein Platin mehr in Lösung befand und lediglich 0,8% der ursprünglichen Palladiumkonzentration noch in Lösung befanden. Das restliche Edelmetall war auf den vorhandenen Träger (Aluminiumoxid und Zeolith) aufgefällt.

## Patentansprüche

1. Ein Tetraalkylammonium-Tetra- oder Hexa-hydroxometallat der Formel (N(Alkyl)₄)_{y}[M(OH)ₓ], wobei
M ein Metall ausgewählt aus der Gruppe bestehend aus Rhodium oder Palladium ist;
Alkyl eine Alkylgruppe mit einem bis 6 Kohlenstoffatomen ist;
Y 1, 2 oder 3 ist; und
X 4 oder 6 ist.

2. Ein Tetraalkylammonium-Tetra- oder Hexa-hydroxometallat nach Anspruch 1, wobei Alkyl eine Alkylgruppe ausgewählt aus der Gruppe bestehend aus Ethyl, Propyl, Isopropyl, n-Butyl, Isobutyl, tert.-Butyl, n-Pentyl, sec-Pentyl, 3-Pentyl, 2-Methylbutyl, Isopentyl, 3-Methylbut-2-yl, 2-Methylbut-2-yl, Neopentyl, n-Hexyl, 3-Methylpentyl, Isohexyl, Neohexyl, 2,3,-Dimethylbutyl oder deren Kombinationen ist.

3. Ein Tetraalkylammonium-Tetra- oder Hexa-hydroxometallat nach Anspruch 1 oder 2, wobei M Palladium und X = 4 oder M Rhodium oder Platin und X = 6 ist.

4. Ein Tetraalkylammonium-Tetra- oder Hexa-hydroxometallat nach Anspruch 1, 2 oder 3, wobei M Palladium und Y = 1, M Rhodium und Y = 3 ist.

5. Verfahren zur Herstellung von Tetraalkylammonium- Tetra- oder Hexahydroxometallaten durch Umsetzung von Hydroxoverbindungen eines Metalls ausgewählt aus der Gruppe bestehend aus Rhodium oder Palladium mit Tetraalkylammoniumhydroxid, optional in Gegenwart eines Reaktionsmediums.

6. Verfahren nach Anspruch 5, wobei ein Tetraalkylammoniumhydroxid oder deren Lösung vorgelegt, Hydroxoverbindungen eines Metalls wie etwa Tetra-oder Hexahydroxosäuren zugegeben und für eine vorbestimmte Reaktionszeit und bei einer in einem vorbestimmten Bereich liegenden Reaktionstemperatur umgesetzt wird.

7. Verfahren nach Anspruch 5, wobei Hydroxoverbindungen eines Metalls wie etwa Tetra-oder Hexahydroxosäuren vorgelegt, Tetraalkylammoniumhydroxid oder deren Lösung zugegeben und für eine vorbestimmte Reaktionszeit und bei einer in einem vorbestimmten Bereich liegenden Reaktionstemperatur umgesetzt wird.

8. Verfahren nach einem oder mehreren der Ansprüche 5 bis 7, wobei das Reaktionsmedium vor Zugabe der Reaktanden vorgelegt wird.

9. Verfahren nach einem oder mehreren der Ansprüche 5 bis 8, wobei das Reaktionsmedium ein polares, protisches Lösemittel ist.

10. Verfahren nach einem oder mehreren der Ansprüche 5 bis 9, wobei das Reaktionsmedium ausgewählt ist aus der Gruppe bestehend aus Wasser, ein oder mehrere Alkohole, eine oder mehrere Säuren und deren Mischungen.

11. Verfahren nach einem oder mehreren der Ansprüche 5 bis 9, wobei das Reaktionsmedium ein Lösemittel ausgewählt aus der Gruppe bestehend aus Wasser, Methanol, Ethanol, Propanol, Isopropanol, Butanol, 1-Methylbutanol, 2-Methylbutanol, tert.- Butanol, Ameisensäure, Essigsäure, Propionsäure und deren Kombinationen ist.

12. Verfahren nach einem oder mehreren der Ansprüche 5 bis 11, wobei das Verfahren bei einer Temperatur von 10°C bis 100°C, insbesondere 15°C bis 80°C oder 20°C bis 50°C oder 20°C bis 40°C durchgeführt wird.

13. Verfahren nach einem oder mehreren der Ansprüche 5 bis 12, wobei 0,5 bis 17 oder 1,7 bis 17, insbesondere 2 bis 6 Moläquivalente Tetraethylammoniumhydroxid pro Mol Hydroxoverbindungen des Metalls wie etwa Tetra-oder Hexahydroxosäuren eingesetzt werden.

14. Verwendung von Tetraalkylammonium-Tetra- oder Hexa-hydroxometallat nach einem oder mehreren der Ansprüche 1 bis 4 oder deren wäßrige Zubereitung als Precursor zur Herstellung heterogener Katalysatoren, Autoabgaskatalysatoren, Materialien für Brennstoffzellen, für den Einsatz in galvanischen Bädern, als Vorprodukt für die Herstellung homogener Katalysatoren oder als homogener Katalysator.

15. Verfahren zur Herstellung eines Autoabgaskatalysators, wobei eine Suspension enthaltend Tetraalkylammonium-Tetra- oder Hexa-hydroxometallat nach einem oder mehreren der Ansprüche 1 bis 4, und mindestens ein Trägermaterial erzeugt wird, durch Umsetzen des Tetraalkylammonium-Tetra- oder Hexa-hydroxometallats das Metall als Hydroxoverbindungen wie etwa Tetra-oder Hexahydroxosäure auf das Trägermaterial gefällt und ein Katalysator-Tragkörper ganz oder teilweise mit dem Trägermaterial beschichtet wird und das auf das Trägermaterial aufgebrachte Tetraalkylammonium-Tetra- oder Hexa-hydroxometallat vor oder nach der Beschichtung des Katalysator-Tragkörpers kalziniert wird.

16. Verfahren nach Anspruch 15, wobei das Trägermaterial ein Metalloxid, insbesondere Aluminiumoxid oder Siliziumdioxid ist.

17. Verfahren nach Anspruch 15 oder 16, wobei der Katalysator-Tragkörper ein Wandflußfilter oder ein Durchflußwabenkörper ist.

18. Verfahren nach einem oder mehreren der Ansprüche 15 bis 17, wobei das Umsetzen des Tetraalkylammonium-Tetra- oder Hexa-hydroxometallats durch Änderung des pH-Wertes erfolgt.

19. Verfahren nach einem oder mehreren der Ansprüche 15 bis 18, wobei das Umsetzen von Tetraalkylammonium-Tetra- oder Hexa-hydroxometallat zum Fällen auf das Trägermaterial durch Änderung des pH-Wertes erfolgt.

## Claims

1. A tetraalkylammonium tetra- or hexa-hydroxometalate of the formula (N(alkyl)₄)_{y} [M(OH)ₓ] wherein
M is a metal selected from the group consisting of rhodium or palladium;
alkyl is an alkyl group with one to 6 carbon atoms;
Y is 1, 2 or 3; and
X is 4 or 6.

2. A tetraalkylammonium tetra- or hexa-hydroxometalate according to claim 1, wherein alkyl is an alkyl group selected from the group consisting of ethyl, propyl, isopropyl, n-butyl, isobutyl, tert-.butyl, n-pentyl, sec-pentyl, 3-pentyl, 2-methylbutyl, isopentyl, 3-methylbut-2-yl, 2-methylbut-2-yl, neopentyl, n-hexyl, 3-methylpentyl, isohexyl, neohexyl, 2,3,-dimethylbutyl, or combinations thereof.

3. A tetraalkylammonium tetra- or hexa-hydroxometalate according to claim 1 or 2, wherein M is palladium and X = 4 or M is rhodium or platinum and X = 6.

4. A tetraalkylammonium tetra- or hexa-hydroxometalate according to claim 1, 2 or 3, wherein M is palladium and Y = 1, M is rhodium and Y = 3.

5. A process for the preparation of tetraalkylammonium tetra- or hexahydroxometalates by reacting hydroxo compounds of a metal selected from the group consisting of rhodium or palladium with tetraalkylammonium hydroxide, optionally in the presence of a reaction medium.

6. The method of claim 5, wherein a tetraalkylammonium hydroxide or solution thereof is provided, hydroxo compounds of a metal such as tetra- or hexahydroxo acids are added and reacted for a predetermined reaction time and at a reaction temperature within a predetermined range.

7. The method of claim 5, wherein hydroxo compounds of a metal such as tetra- or hexahydroxo acids are provided, tetraalkylammonium hydroxide or its solution is added and reacted for a predetermined reaction time and at a reaction temperature within a predetermined range.

8. The method of one or more of claims 5 to 7, wherein the reaction medium is provided prior to addition of the reactants.

9. The method of one or more of claims 5 to 8, wherein the reaction medium is a polar protic solvent.

10. The method of one or more of claims 5 to 9, wherein the reaction medium is selected from the group consisting of water, one or more alcohols, one or more acids and mixtures thereof.

11. The method of one or more of claims 5 to 9, wherein the reaction medium is a solvent selected from the group consisting of water, methanol, ethanol, propanol, isopropanol, butanol, 1-methylbutanol, 2-methylbutanol, tert-butanol, formic acid, acetic acid, propionic acid, and combinations thereof.

12. A process according to one or more of claims 5 to 11, wherein the process is carried out at a temperature of 10°C to 100°C, in particular 15°C to 80°C or 20°C to 50°C or 20°C to 40°C.

13. The method according to one or more of claims 5 to 12, wherein 0.5 to 17 or 1.7 to 17, in particular 2 to 6 molar equivalents of tetraethylammonium hydroxide per mole of hydroxo compounds of the metal such as tetra- or hexahydroxo acids are used.

14. The use of tetraalkylammonium tetra- or hexa-hydroxometalate according to one or more of claims 1 to 4 or an aqueous preparation thereof as a precursor for the preparation of heterogeneous catalysts, automotive exhaust gas catalysts, materials for fuel cells, for use in electroplating baths, as a precursor for the preparation of homogeneous catalysts or as a homogeneous catalyst.

15. A process for the preparation of an automotive exhaust catalyst, wherein a suspension comprising tetraalkylammonium tetra- or hexa-hydroxometalate according to one or more of claims 1 to 4, and at least one support material is produced, by reacting the tetraalkylammonium tetra- or hexa-hydroxometallate, the metal is precipitated onto the support material as hydroxo compounds such as tetra- or hexahydroxo acid, and a catalyst support body is coated wholly or partially with the support material, and the tetraalkylammonium tetra- or hexa-hydroxometallate applied to the support material is calcined before or after coating the catalyst support body.

16. The method according to claim 15, wherein the carrier material is a metal oxide, in particular aluminium oxide or silicon dioxide.

17. The method of claim 15 or 16, wherein the catalyst support body is a wall flow filter or a flow-through-honeycomb body.

18. The method of one or more of claims 15 to 17, wherein the reacting of the tetraalkylammonium tetra- or hexa-hydroxometalate is by changing the pH.

19. The method of one or more of claims 15 to 18, wherein reacting tetraalkylammonium tetra- or hexa-hydroxometalate to precipitate onto the support material is done by changing the pH.

## Revendications

1. Un tétra- ou hexa-hydroxométallate de tétraalkylammonium de formule (N(alkyle)₄)_{y} [M(OH)ₓ], dans laquelle
M est un métal choisi dans le groupe constitué par le rhodium ou le palladium ;
alkyle est un groupe alkyle ayant de un à six atomes de carbone ;
Y est égal à 1, 2 ou 3 ; et
X est 4 ou 6.

2. Tétra- ou hexa-hydroxométallate de tétraalkylammonium selon la revendication 1, dans lequel alkyle représente un groupe alkyle choisi dans le groupe constitué par éthyle, propyle, isopropyle, n-butyle, isobutyle, tert.butyle, n-pentyle, sec-pentyle, 3-pentyle, 2-méthylbutyle, isopentyle, 3-méthylbut-2-yle, 2-méthylbut-2-yle, néopentyle, n-hexyle, 3-méthylpentyle, isohexyle, néohexyle, 2,3,-diméthylbutyle ou leurs combinaisons.

3. Un tétra- ou hexa-hydroxométallate de tétraalkylammonium selon la revendication 1 ou 2, dans lequel M est le palladium et X = 4 ou M est le rhodium ou le platine et X = 6.

4. Un tétra- ou hexa-hydroxométallate de tétraalkylammonium selon la revendication 1, 2 ou 3, dans lequel M est le palladium et Y = 1, M est le rhodium et Y = 3.

5. Procédé de préparation de tétra- ou hexa-hydroxométallates de tétraalkylammonium par réaction de composés hydroxylés d'un métal choisi dans le groupe constitué par le rhodium ou le palladium avec de l'hydroxyde de tétraalkylammonium, éventuellement en présence d'un milieu réactionnel.

6. Procédé selon la revendication 5, dans lequel on dispose d'un hydroxyde de tétraalkylammonium ou d'une solution de celui-ci, on ajoute des composés hydroxylés d'un métal, tels que des tétra- ou hexahydroxyacides, et on fait réagir pendant un temps de réaction prédéterminé et à une température de réaction se situant dans une plage prédéterminée.

7. Procédé selon la revendication 5, dans lequel des composés hydroxylés d'un métal, tels que des tétra- ou hexahydroxyacides, sont fournis, de l'hydroxyde de tétraalkylammonium ou sa solution est ajouté et mis à réagir pendant un temps de réaction prédéterminé et à une température de réaction située dans une plage prédéterminée.

8. Procédé selon l'une ou plusieurs des revendications 5 à 7, dans lequel le milieu réactionnel est présenté avant l'addition des réactifs.

9. Procédé selon une ou plusieurs des revendications 5 à 8, dans lequel le milieu réactionnel est un solvant polaire protique.

10. Procédé selon une ou plusieurs des revendications 5 à 9, dans lequel le milieu réactionnel est choisi dans le groupe constitué par l'eau, un ou plusieurs alcools, un ou plusieurs acides et leurs mélanges.

11. Procédé selon une ou plusieurs des revendications 5 à 9, dans lequel le milieu réactionnel est un solvant choisi dans le groupe constitué par l'eau, le méthanol, l'éthanol, le propanol, l'isopropanol, le butanol, le 1-méthylbutanol, le 2-méthylbutanol, le tert-butanol, l'acide formique, l'acide acétique, l'acide propionique et leurs combinaisons.

12. Procédé selon une ou plusieurs des revendications 5 à 11, dans lequel le procédé est mis en oeuvre à une température de 10°C à 100°C, en particulier de 15°C à 80°C ou de 20°C à 50°C ou de 20°C à 40°C.

13. Procédé selon une ou plusieurs des revendications 5 à 12, dans lequel on utilise 0,5 à 17 ou 1,7 à 17, en particulier 2 à 6 équivalents molaires d'hydroxyde de tétraéthylammonium par mole de composés hydroxylés du métal, tels que des tétra- ou hexahydroxyacides.

14. Utilisation de tétra- ou hexa-hydroxométallate de tétraalkylammonium selon une ou plusieurs des revendications 1 à 4 ou de leur préparation aqueuse comme précurseur pour la production de catalyseurs hétérogènes, de catalyseurs de gaz d'échappement d'automobiles, de matériaux pour piles à combustible, pour l'utilisation dans des bains galvaniques, comme précurseur pour la production de catalyseurs homogènes ou comme catalyseur homogène.

15. Procédé de préparation d'un catalyseur pour gaz d'échappement automobile, dans lequel on prépare une suspension contenant du tétra- ou hexa-hydroxométalate de tétraalkylammonium selon une ou plusieurs des revendications 1 à 4, et au moins un matériau support, par réaction du tétra- ou hexa-hydroxométalate de tétraalkylammonium, le métal est précipité sur le matériau de support sous forme de composés hydroxylés tels que l'acide tétra- ou hexahydroxo que et un corps de support de catalyseur est revêtu entièrement ou partiellement du matériau de support et le tétra- ou hexa-hydroxométalate de tétraalkylammonium appliqué sur le matériau de support est calciné avant ou après le revêtement du corps de support de catalyseur.

16. Procédé selon la revendication 15, dans lequel le matériau de support est un oxyde métallique, en particulier de l'alumine ou de la silice.

17. Procédé selon la revendication 15 ou 16, dans lequel le corps de support du catalyseur est un filtre à écoulement mural ou un corps en nid d'abeilles à écoulement continu.

18. Procédé selon l'une ou plusieurs des revendications 15 à 17, dans lequel la réaction du tétra- ou hexa-hydroxométallate de tétraalkylammonium est réalisée par modification du pH.

19. Procédé selon l'une ou plusieurs des revendications 15 à 18, dans lequel la réaction du tétra- ou hexa-hydroxométallate de tétraalkylammonium pour la précipitation sur le matériau support est réalisée par modification du pH.
